# EUROPÄISCHE PATENTSCHRIFT

(11) **EP 3 799 580 B1**
(45) Veröffentlichungstag und Bekanntmachung des Hinweises auf die Patenterteilung: **12.01.2022**
(21) Anmeldenummer: 19787203.9
(22) Anmeldetag: 10.10.2019
(51) Int. Cl.: B07C 5/34

(54) **VERFAHREN ZUR GEZIELTEN AUSWAHL EINES SENSORS ZUR SENSORBASIERTEN SORTIERUNG EINES MATERIALGEMISCHES DURCH SIMULATION DER SENSORBASIERTEN SORTIERUNG DES MATERIALGEMISCHES**
MÉTHODE POUR LA SÉLECTION D'UN CAPTEUR DESTINÉ AU TRI D'UN MÉLANGE DE MATÉRIAUX BASÉE SUR LA SIMULATION DU FONCTIONNEMENT D'UN TEL CAPTEUR
PROCÉDÉ DE SÉLECTION AJUSTÉE D'UN CAPTEUR POUR LE TRI D'UN MÉLANGE MATÉRIAUX PAR CAPTEUR UTILISANT UNE SIMULATION DE TRI DE CET MÉLANGE MATÉRIAUX PAR CAPTEUR

(30) Priorität: 12.10.2018 DE 102018217548
(43) Veröffentlichungstag der Anmeldung: 07.04.2021
(73) Patentinhaber: Helmholtz-Zentrum Dresden - Rossendorf e.V., 01328 Dresden (DE)
(72) Erfinder: KERN, Marius, 01328 Dresden (DE); TUSA, Laura, 01328 Dresden (DE); GUTZMER, Jens, Dresden - Rossendorf e.V. Bautzner Landstraße 400 Dresden 01328 (DE); VAN DEN BOOGAART, Karl Gerald, 01328 Dresden (DE)
(74) Vertreter: Loock, Jan Pieter
(86) Internationale Anmeldenummer: PCT/EP2019/077524
(87) Internationale Veröffentlichungsnummer: WO 2020/074665

(56) Entgegenhaltungen:
- US-A1- 2013 292 307

## Beschreibung

### Hintergrund der Erfindung

Die Erfindung betrifft ein Verfahren zur gezielten Auswahl eines Sensors zur sensorbasierten Sortierung von Materialgemischen, z.B. von partikulären Materialgemischen mit potentiell mehrphasigen Partikeln, wie beispielsweise aus US-A-2013/0292307 bekannt.

Bei der Gewinnung oder Rückgewinnung von Wertstoffen aus heterogenen Materialgemischen wird häufig eine sensorbasierte Sortierung zur Abtrennung von wertstoffarmen Komponenten des Materialgemisches und zur Anreicherung von wertstoffreichen Komponenten des Materialgemisches genutzt. Die sensorbasierte Sortierung wird erfolgreich in der Bergbaubranche, beispielsweise bei der Wertstoffgewinnung aus Gestein, in der Nahrungsmittelindustrie oder auch in der Abfall- oder Recyclingwirtschaft beim Trennen von Wertstoffen eingesetzt. Basis für eine funktionierende sensorbasierte Sortierung ist, dass der zur Sortierung eingesetzte Sensor in der Lage ist, eine bestimmte Materialeigenschaft schnell und präzise zu erkennen.

Die zu detektierende Materialeigenschaft ist dabei nicht immer das direkte Vorhandensein des Wertstoffes. Es ist auch denkbar, dass Materialeigenschaften physikalischer oder chemischer Art detektiert werden, die nur indirekt mit dem Vorhandensein des Wertstoffes verknüpft sind. In jedem Fall muss für den erfolgreichen Einsatz des sensorbasierten Sortierverfahrens ein Sensor aus einer Vielzahl von am Markt verfügbaren Sensoren ausgewählt werden, welcher dafür geeignet ist, wertstoffhaltiges und wertstofffreies Material anhand von einer einzigen Materialeigenschaft voneinander zu unterscheiden. Dieses Auswählen eines Sensors wird nach dem Stand der Technik durch "Trial-and-Error-Versuche" durchgeführt.

Dazu wird zunächst ein Sensor ausgewählt, der die Unterscheidung zwischen wertstoffhaltig und wertstofffrei mutmaßlich vollziehen kann. Diese Abschätzung basiert oft auf optischer Begutachtung und wenigen, beispielsweise geochemischen oder mineralogischen, Analysen.

Bei einem Versuch mit etwa 100 einzelnen Proben eines Materialgemisches, beispielsweise Gesteinsproben mit Korngrößen von jeweils 10 bis 100 mm, wird das Material mit dem ausgewählten Sensor auf einem Versuchsstand sortiert. Anschließend wird der Erfolg der Trennung anhand von chemischen Analysen ermittelt. Sollte sich im Konzentrat, also dem Teil des Materialgemisches, welches von dem ausgewählten Sensor als wertstoffhaltig eingestuft wurde, nun ein erheblicher Teil des Wertstoffes befinden, werden die Versuche als erfolgreich eingestuft. Nach weiteren Versuchen in einem größeren Maßstab könnte der ausgewählte in einem kommerziellen Betrieb Anwendung finden und in ein Sortierungsverfahren implementiert werden.

Bei dem Auswahlverfahren nach dem Stand der Technik werden nur in geringem Maße Daten bezüglich der chemischen und physikalischen Materialeigenschaften des zu sortierenden Materials erhoben. Daraus ergeben sich einige Nachteile. So bleibt mangels Messdaten teilweise unklar, aus welchem Grund eine Entscheidung zum Aussortieren einer Probe oder zum Einsortieren einer Probe in das Konzentrat getroffen wird. Weiterhin ist es nicht möglich zu sagen, ob der gewählte Sensor sein volles Potential ausnutzt oder ob man mit leicht veränderten Einstellungen am Sensor möglicherweise ein besseres Sortierergebnis erlangen würde. Ferner wird aus Kostengründen typischerweise nur ein einziger Sensor getestet. Es ist daher nach dem Auswahlverfahren nach dem Stand der Technik nicht auszuschließen, dass mit einem anderen Sensor bessere Messergebnisse möglich wären. Bei den gezeigten Nachteilen des Standes der Technik ist es ersichtlich, dass keine zuverlässige qualifizierte Kosten-Nutzen-Rechnung möglich ist.

### Offenbarung der Erfindung

Es ist eine Aufgabe der vorliegenden Erfindung die im Zusammenhang mit dem Stand der Technik genannten Nachteile zu vermeiden und ein Verfahren bereitzustellen, welches geeignet ist, vorherzusagen, welcher Sensor anhand von welchen physikalischen oder chemischen Materialeigenschaften zu welchem Sortierergebnis führt, welches ferner in der Lage ist, Auskunft darüber zu geben, ob ein Sensor wirklich sein volles Potential nutzt und welches eine Aussage darüber treffen lässt, welche Materialeigenschaften die wertstoffhaltigen Bestandteile und die wertstofffreien Bestandteile des Materialgemisches nach der Sortierung aufweisen. Dies ermöglicht eine belastbare Kosten-Nutzen-Rechnung des Verfahrens und damit eine wirtschaftlich sinnvolle sensorbasierte Sortierung des Materialgemisches.

Die Aufgabe der vorliegenden Erfindung wird gelöst durch ein Verfahren zur Auswahl eines Sensors (z.B. eines einzigen Sensors oder mehrerer Sensoren) aus einer Mehrzahl von Sensoren zur sensorbasierten Sortierung eines Materialgemischs anhand einer Materialeigenschaft zur Konzentration eines Wertstoffes, wobei in einem ersten Schritt aus dem Materialgemisch repräsentative Einzelproben gewonnen werden, in einem zweiten Schritt Materialeigenschaften der Einzelproben gemessen werden, in einem dritten Schritt auf Grundlage der gemessenen Materialeigenschaften eine Mehrzahl von simulierten Sortierungen anhand jeweils unterschiedlicher Materialeigenschaften simuliert wird, in einem vierten Schritt eine simulierte Sortierung mit einer hohen Wertstoffkonzentration aus der Mehrzahl von simulierten Sortierungen ausgewählt wird, in einem fünften Schritt ein Sensor zur Messung der Materialeigenschaft ausgewählt wird, welcher geeignet ist, mindestens eine der Materialeigenschaften, welche für die ausgewählte simulierte Sortierung genutzt wurde, zu messen.

Damit ist es vorteilhaft möglich, den Zusammenhang zwischen den physikalischen und chemischen Materialeigenschaften und dem Sortierergebnis herzustellen und somit auszuloten, ob das gesamte Potential eines Sensors genutzt wird.

Materialeigenschaften im Sinne der vorliegenden Erfindung sind physikalische oder chemische Eigenschaften des Materials. Dies können beispielsweise ein typisches optisches Absorptions- oder Emissionsspektrum, ein typisches Absorptions- oder Emissionsspektrum im Röntgenbereich oder auch magnetische Eigenschaften sein. Die Materialeigenschaften können dabei direkt oder auch indirekt mit dem Vorhandensein oder Nicht-Vorhandensein eines Wertstoffes im Material zusammenhängen. Ist es möglich, den Wertstoffgehalt einer Probe direkt als Materialeigenschaft zu messen, so ist diese Materialeigenschaft natürlich die geeignetste Materialeigenschaft, um Aussagen bezüglich des Wertstoffgehaltes einer Einzelprobe zu treffen. Mitunter ist es allerdings nicht möglich, den Wertstoffgehalt einer Einzelprobe als Materialeigenschaft direkt zu messen. In einem solchen Fall muss von einer anderen Materialeigenschaft auf den Wertstoffgehalt einer Einzelprobe geschlossen werden.

Die Sortierung des Materialgemisches bedeutet im Sinne der vorliegenden Erfindung folgenden Ablauf: Das Materialgemisch wird in Einzelproben aufgeteilt. Die Einzelproben werden dann jeweils anhand ihrer Materialeigenschaften als wertstoffhaltig oder wertstofffrei bewertet. Dabei bedeutet wertstoffhaltig, dass die Einzelprobe den Wertstoff in einer Konzentration aufweist, welche oberhalb eines bestimmten Schwellenwertes liegt. Wertstofffrei bedeutet, dass die Einzelprobe den Wertstoff in einer Konzentration aufweist, welche unterhalb eines bestimmten Schwellenwertes liegt. Die wertstoffhaltigen Einzelproben werden von den wertstofffreien Einzelproben getrennt, wobei erstere nach der Sortierung als Konzentrat des Wertstoffes zur Verfügung stehen und letztere das sogenannte Haldenmaterial bilden.

Die Messung der Materialeigenschaften im zweiten Schritt ist eine Messung einer Vielzahl unterschiedlicher Materialeigenschaften an jeder einzelnen Einzelprobe. Denkbar ist, dass die Messung der Materialeigenschaften im zweiten Schritt unmittelbar und/oder mittelbar durchgeführt wird. Dabei bedeutet unmittelbar gemessen im Sinne der vorliegenden Erfindung, dass eine chemische und/oder physikalische Materialeigenschaft direkt gemessen wird. Dies kann beispielsweise die Messung eines optischen Spektrums mit Hilfe einer Spektralanalyse sein. Mittelbar gemessen im Sinne der vorliegenden Erfindung bedeutet, dass die Materialeigenschaften auf Grund der Messung eines mit der Materialeigenschaft verknüpften Materialparameters zugeordnet werden. So kann beispielsweise anhand einer gemessenen chemischen Signatur, also des gemessenen Materialparameters, auf das Mineral geschlossen werden, an welchem der Materialparameter gemessen wurde. Anschließend können dem Mineral bekannte Materialeigenschaften zugeordnet werden. Denkbar ist, dass dem Mineral beispielsweise Materialeigenschaften wie Ferromagnetismus, Farbe oder Absorptionsmaxima zugeordnet werden. Mit anderen Worten wird beim mittelbaren Messen einer Materialeigenschaft die Materialeigenschaft auf Grundlage der Messung einer anderen Materialeigenschaft zugeordnet. Man könnte auch sagen, dass durch das mittelbare Messen von Materialeigenschaften ein Satz von unterschiedlichen Materialeigenschaften durch das zuordnen von Materialeigenschaften zu unmittelbar gemessenen Materialeigenschaften vervollständigt wird. Anhand der gemessenen Materialeigenschaften aus der Vielzahl der Materialeigenschaften werden im dritten Schritt Sortierungen simuliert. Denkbar wäre beispielsweise, dass eine erste Sortierung nach dem Vorhandensein eines ersten optischen Emissionsmaximums (beispielsweise eine rote Farbgebung der Einzelproben), eine zweite Sortierung nach dem Vorhandensein eines zweiten optischen Emissionsmaximums (beispielsweise eine grüne Farbgebung der Einzelproben), eine dritte Sortierung nach vorhandenem Ferromagnetismus und eine vierte Sortierung nach einem Absorptionsmaximum im Röntgenbereich simuliert wird. Es sind Sortierungen anhand einer Vielzahl von Materialeigenschaften denkbar. Beispielsweise erwähnt seien hier Sortierungen anhand von Absorptionseigenschaften im kurzwellen-Infrarotbereich, also zwischen 1230 nm Wellenlänge und 1250 nm Wellenlänge und Sortierungen anhand von Messungen mit einem XRF-Sensor, also Eisengehalten. Im vierten Schritt werden nun aus den simulierten Sortierungen eine oder mehrere Sortierungen ausgewählt, welche zu einer hohen (simulierten) Konzentration des Wertstoffes geführt haben. Um beim Beispiel von oben zu bleiben, könnte festgestellt werden, dass bei der zweiten Sortierung, nämlich nach der nach der grünen Farbgebung, eine besonders hohe Konzentration des Wertstoffes vorliegt. Im fünften Schritt wird alsdann entsprechend der Materialeigenschaft, welche Grundlage der ausgewählten Sortierung war, ein passender Sensor ausgewählt. Im Beispiel von oben wäre dies ein Sensor zur Detektion eines grünen Farbtones. Denkbar ist, dass die Simulationen für verschiedene Schwellenwerte durchgeführt werden.

Denkbar ist, dass der zweite Schritt computerimplementiert durchgeführt wird. Denkbar ist, dass der dritte Schritt computerimplementiert durchgeführt wird. Denkbar ist ferner, dass der vierte Schritt computerimplementiert durchgeführt wird. Schließlich ist denkbar, dass der fünfte Schritt computerimplementiert durchgeführt wird.

Vorteilhafte Ausgestaltungen und Weiterbildungen der Erfindung sind den Unteransprüchen, sowie der Beschreibung unter Bezugnahme auf die Zeichnungen entnehmbar.

Gemäß einer bevorzugten Ausführungsform der vorliegenden Erfindung ist vorgesehen, dass die Einzelproben anhand von Messungen mit dem ausgewählten Sensor sortiert werden und überprüft wird, ob die anhand der Messungen mit dem ausgewählten Sensor erfolgte Sortierung der Einzelproben mit der ausgewählten simulierten Sortierung übereinstimmt. Damit ist es auf vorteilhafte Weise möglich, anhand der Einzelproben, deren gemessenen Materialeigenschaften die Grundlage der ausgewählten simulierten Sortierung im dritten Schritt sind, zu überprüfen, ob der ausgewählte Sensor tatsächlich so sortiert, wie von der Simulation vorhergesagt. Im günstigsten Fall stimmen Simulation und erfolgte Sortierung überein, das heißt der Sensor ermittelt die Materialeigenschaft, welche der ausgewählten simulierten Sortierung zu Grunde lag, der jeweiligen Einzelprobe korrekt und es erfolgt eine Sortierung der Einzelproben mit dem ausgewählten Sensor, wie sie simuliert wurde. Abweichungen, wie beispielsweise mangelhafte Genauigkeit des Sensors oder Probleme mit der Detektierbarkeit der Materialeigenschaft, können so direkt erkannt und gegebenenfalls abgestellt werden.

Gemäß einer bevorzugten weiteren Ausführungsform der vorliegenden Erfindung ist vorgesehen, dass wenn die anhand der Messungen mit dem ausgewählten Sensor erfolgte Sortierung der Einzelproben mit der simulierten Sortierung nicht übereinstimmt, Einstellungen des ausgewählten Sensors angepasst werden, um die Sortierung der Einzelproben anhand von Messungen mit dem ausgewählten Sensor mit der ausgewählten simulierten Sortierung zur Übereinstimmung zu bringen. Dies ermöglicht, Abweichungen beim Detektieren der Materialeigenschaft auszugleichen. Der ausgewählte Sensor kann so besser an die zu detektierende Materialeigenschaft angepasst und damit die sensorbasierte Sortierung optimiert werden. Denkbar ist, dass nach dem Anpassen der Einstellungen des Sensors erneut die Einzelproben anhand von Messungen mit dem ausgewählten Sensor mit den angepassten Eigenschaften sortiert werden und überprüft wird, ob die anhand der Messungen mit dem ausgewählten Sensor mit den angepassten Eigenschaften erfolgte Sortierung der Einzelproben mit der ausgewählten simulierten Sortierung übereinstimmt. Dies ermöglicht eine Kontrolle der Änderung der Einstellungen des Sensors. Denkbar ist ferner, dass die Einstellungen des Sensors so lange angepasst und durch eine erneute Sortierung überprüft werden, bis die Einstellungen des Sensors optimal eingestellt sind.

Gemäß einer bevorzugten weiteren Ausführungsform der vorliegenden Erfindung ist vorgesehen, dass wenn es nicht möglich ist, die Sortierung der Einzelproben anhand von Messungen mit dem ausgewählten Sensor mit der ausgewählte simulierten Sortierung in Übereinstimmung zu bringen, der vierte Schritt und der fünfte Schritt wiederholt werden und eine andere Materialeigenschaft und/oder ein anderer Sensor gewählt wird. Dies ermöglicht die Anpassung der Auswahl des Sensors und/oder der zu detektierenden Materialeigenschaft in dem Falle, dass sich der experimentelle Nachweis der simulierten Sortierung nicht erbringen lässt, also beispielsweise nicht behebbare Probleme mit dem ursprünglich ausgewählten Sensor vorliegen.

Vorzugsweise ist vorgesehen, dass die Einzelproben anhand von Messungen mit dem anderen Sensor respektive auf Grundlage der Messung der anderen Materialeigenschaft sortiert werden und überprüft wird, ob die anhand der Messungen mit dem anderen Sensor respektive auf Grundlage der Messung der anderen Materialeigenschaft erfolgte Sortierung der Einzelproben mit der ausgewählten simulierten Sortierung respektive mit der simulierten Sortierung auf Grundlage anderen Materialeigenschaft übereinstimmt.

Besonders bevorzugt ist vorgesehen, dass wenn die anhand der Messungen mit dem anderen Sensor erfolgte Sortierung der Einzelproben mit der simulierten Sortierung nicht übereinstimmt respektive wenn die anhand der Messungen der anderen Materialeigenschaft erfolgte Sortierung der Einzelproben mit der simulierten Sortierung auf Grundlage der anderen Materialeigenschaft nicht übereinstimmt, Einstellungen des ausgewählten Sensors respektive des anderen Sensors angepasst werden um die Sortierung der Einzelproben anhand von Messungen mit dem anderen Sensor respektive die Sortierung auf Grundlage der Messung der anderen Materialeigenschaft mit der ausgewählten simulierten Sortierung respektive mit der simulierten Sortierung auf Grundlage der anderen Materialeigenschaft zur Übereinstimmung zu bringen.

Gemäß einer bevorzugten weiteren Ausführungsform der vorliegenden Erfindung ist vorgesehen, dass die Einzelproben in einem vorbereitenden Schritt zwischen dem ersten Schritt und dem zweiten Schritt mechanisch bearbeitet werden, wobei an den Einzelproben bevorzugt eine plane Seite hergestellt wird, wobei die Einzelproben besonders bevorzugt zu Quadern gesägt werden. Dies ermöglicht eine zuverlässige und genaue Messung der Materialeigenschaften. Weiterhin wird, besonders durch Sägen der Einzelproben in Quader, ein höherer Automatisierungsgrad des Verfahrens ermöglicht. Denkbar ist, dass die Einzelproben zwischen dem ersten Schritt und dem zweiten Schritt gereinigt und/oder poliert werden. Denkbar ist, dass die Quader Kantenlängen von weniger als einem Zentimeter und bis zu mehreren Zentimetern aufweisen. Bevorzugt weisen die Quader eine Länge von 1 cm bis 20 cm, besonders bevorzugt von 2,5 cm bis 10 cm und insbesondere von circa 5 cm auf. Bevorzugt weisen die Quader eine Breite von 0,5 cm bis 10 cm, besonders bevorzugt von 1 cm bis 5 cm und insbesondere von circa 2,5 cm auf. Bevorzugt weisen die Quader eine Tiefe von 0,1 cm bis 5 cm, besonders bevorzugt von 0,2 cm bis 2 cm und insbesondere von circa 0,5 cm auf.

Denkbar ist, dass die Einzelproben zwischen dem ersten Schritt und dem zweiten Schritt nicht poliert werden. Denkbar ist, dass die Einzelproben zwischen dem ersten Schritt und dem zweiten Schritt gewaschen werden. Denkbar dazu ist, dass die die Einzelproben zwischen dem ersten Schritt und dem zweiten Schritt gewaschen und gesägt werden. Denkbar ist aber auch, dass die Einzelproben zwischen dem ersten Schritt weder gewaschen noch gesägt werden. Dies wird durch die Verwendung von modernen Messeverfahren und Messvorrichtungen ermöglicht. Dazu ist beispielsweise vorstellbar, dass im zweiten Schritt Materialeigeschaften mit Hilfe eines Computertomographen gemessen werden. Dazu ist es möglich, dass der Computertomograph mehrere Energieniveaus misst.

Gemäß einer bevorzugten weiteren Ausführungsform der vorliegenden Erfindung ist vorgesehen, dass die Einzelproben jeweils mit der planen Seite auf ein Glasplättchen aufgeklebt werden, wobei die Einzelproben jeweils parallel zum Glasplättchen in einen glasplättchenseitigen Anschliff und eine Restprobe getrennt werden, wobei der Anschliff vorzugsweise als Gesteinsanschliff präpariert wird. Zu jeder Einzelprobe ergibt sich damit ein Set aus einer Restprobe und einem Anschliff. Das Trennen in Anschliff und Restprobe ermöglicht zum einen eine hoch präzise und belastbare Messung der Materialeigenschaften am Anschliff und zum anderen ein Durchführen der Sortierung der Einzelproben mit dem ausgewählten Sensor anhand der Restproben. Denkbar ist aber auch, dass die Messung der Materialeigenschaft direkt an der Einzelprobe oder an der Restprobe durchgeführt wird. Denkbar ist dazu, dass die Einzelprobe und/oder die Restprobe vor der Messung aufgebrochen wird. Denkbar ist dazu aber auch, dass die Messung an der unzerkleinerten Einzelprobe durchgeführt wird.

Vorzugsweise ist dazu vorgesehen, dass das Trennen in Anschliff und Restprobe in einem Abstand von 5 µm bis 2000 µm, bevorzugt von 50 µm bis 1500 µm, besonders bevorzugt von 100 µm bis 800 µm und insbesondere von 400 µm bis 500 µm µm von dem Glasplättchen durchgeführt wird.

Gemäß einer bevorzugten weiteren Ausführungsform der vorliegenden Erfindung ist vorgesehen, dass im zweiten Schritt zum Messen der Materialeigenschaften der Einzelproben elektronenmikroskopische Untersuchungsmethoden verwendet werden, wobei vorzugsweise ein Rasterelektronenmikroskop (SEM) mit einem energiedispersiven Spektrometer verwendet wird, wobei besonders bevorzugt als energiedispersives Spektrometer ein energiedispersives Röntgenspektrometer verwendet wird, wobei insbesondere zum Messen der Materialeigenschaften der Einzelproben ein System zum automatisierten Analysieren von mineralogischen Proben, beispielsweise ein Mineral Liberation Analyzer, verwendet wird. Elektronenmikroskopische Untersuchungen sind sehr genau und eignen sich hervorragend für die Materialanalyse. Denkbar sind auch andere elektronenmikroskopische Untersuchungsmethoden, wie zum Beispiel Elektronenenergieverlust-Spektroskopie (EELS), Auger-Elektronen-Spektroskopie (AES), Elektronenrückstreubeugung (EBSD), jeweils in Verbindung mit einem Rasterelektronenmikroskop (SEM), einem Transmissionselektronenmikroskop (TEM), einem Rastertransmissionselektronenmikroskop (STEM) oder einem Focused-Ion-Beam-Mikroskop (FIB), oder auch Röntgen-Mikofluoreszenzanalyse (µRFA) mit einem entsprechenden Röntgen-Mikofluoreszenz-Spektrometer, beispielsweise einem Bruker M4 Tornado. Denkbar ist, dass beim zweiten Schritt das Messen der Materialeigenschaften zumindest teilweise automatisiert durchgeführt wird. Denkbar ist weiterhin, dass ein QEMSCAN-System oder ein TIMA-X-System oder ein Mineralogic-System oder ein IncaMineral-System verwendet wird.

Gemäß einer bevorzugten weiteren Ausführungsform der vorliegenden Erfindung ist vorgesehen, dass im zweiten Schritt mit durch die Messungen der Materialeigenschaften der Einzelproben gewonnene Daten für die Einzelproben jeweils eine zweidimensionale Mineralverteilungskarte der Einzelprobe erstellt wird, wobei bevorzugt in den Mineralverteilungskarten jeweils Informationen zu Mineralgehalt der Einzelprobe und/oder zur Korngröße von Mineralen der Einzelprobe und/oder zu Verwachsungen der Minerale der Einzelprobe hinterlegt werden. Denkbar ist auch, dass im zweiten Schritt mit durch die Messungen der Materialeigenschaften der Einzelproben gewonnene Daten für die Einzelproben jeweils eine dreidimensionale Mineralverteilungskarte der Einzelprobe erstellt wird, wobei bevorzugt in den Mineralverteilungskarten jeweils Informationen zu Mineralgehalt der Einzelprobe und/oder zur Korngröße von Mineralen der Einzelprobe und/oder zu Verwachsungen der Minerale der Einzelprobe hinterlegt werden.

Mineralverteilungskarten im Sinne der vorliegenden Erfindung sind Falschfarbenbilder welche aus Datensätzen erstellt werden, die räumliche und chemische und/oder mineralogische Informationen beinhalten. Ein Datensatz besteht beispielswiese aus einer Position (dargestellt durch x/y-Koordinaten in einer zweidimensionale Ebene oder durch x/y/z-Koordinaten in einem dreidimensionaler Raum). Für diese Position sind chemische und/oder mineralogische Messinformationen und/oder Messwerte und/oder Materialeigenschaften hinterlegt, welchen mit einer MLA-Software ein Mineral zugeordnet werden. Durch das Vorhandensein einer Vielzahl von Positionen mit zugeordneten Mineralen lässt sich eine Mineralverteilungskarte generieren.

Auf Grundlage der Mineralverteilungskarten lässt sich auf eine Vielzahl von sensierbaren Materialeigenschaften schließen. So lassen sich auf Grundlage der in der Mineralverteilungskarte enthaltenen Minerale mittelbar weitere Materialeigenschaften der Einzelprobe ermitteln. Sind Materialeigenschaften eines Minerals bekannt, welche nicht unmittelbar gemessen wurden, so können diese Materialeigenschaften dennoch der Einzelprobe zugeordnet werden. Ein teilweise gemessener Satz von Materialeigenschaften kann so vervollständigt oder zumindest ergänzt und dem Verfahren zugänglich gemacht werden. Aus einer Mineralverteilungskarte können z.B. physikalisch detektierbare Eigenschaften wie magnetische Suszeptibilität, Dichte oder Röntgenschwächung, und/oder spektral detektierbare Eigenschaften wie Farbe, Absorptionsbanden, etc. ermittelt werden. Weiterhin enthält die örtliche Verteilung der Mineralverteilung Informationen über den Wertstoffgehalt der Einzelprobe. Es kann z.B. vorgesehen sein, die Wertstoffkonzentration selbst aus einer solchen Mineralverteilungskarte zu ermitteln. Denkbar ist jedoch auch, dass eine Elementverteilungskarte statt oder zusätzlich zu der Mineralverteilungskarte erstellt wird. Denkbar ist weiterhin, dass statt oder zusätzlich zu der Mineralverteilungskarte und/oder der Elementverteilungskarte eine Dichteverteilungskarte erstellt wird. Die Elementverteilungskarte und die Dichteverteilungskarte liefern auf vorteilhafte Weise Informationen über Materialeigenschaften, welche beispielsweise in der Nahrungsmittelindustrie oder der Abfallwirtschaft bei der Sortierung verwendet werden.

Gemäß einer bevorzugten weiteren Ausführungsform der vorliegenden Erfindung ist vorgesehen, dass die Messung der Einzelproben nach Anspruch 2 an den jeweiligen Restproben durchgeführt wird. Die Restproben sind robust, so dass die Messung der Einzelproben nach Anspruch 2 auch mehrfach ohne die Gefahr der Schädigung der Restproben durchgeführt werden kann.

Gemäß einer bevorzugten weiteren Ausführungsform der vorliegenden Erfindung ist vorgesehen, dass als Sensor der Mehrzahl von Sensoren zumindest ein Röntgensensor, vorzugsweise ein X-Ray Transmission-Sensor oder ein Dual-Energy X-Ray Transmission-Sensor und/oder ein Röntgenfluoreszenz-Sensor, und/oder ein Farbsensor und/oder ein Infrarot-Sensor und/oder ein Magnet-Sensor und/oder ein Sensor zur Bestimmung elektrischer Felder verwendet wird. Denkbar ist auch, dass als Sensor ein Sensor zur Bestimmung laserinduzierter Fluoreszenz, ein Sensor zur laserinduzierten Plasmaspektroskopie, ein IR-Sensor, ein Sensor für Nah-Infrarot, ein Terahertz-Sensor, ein Radar-Sensor, eine Time-Of-Flight-Kamera, oder ein Sensor zur Raman-Spektroskopie als Sensor verwendet wird. Die genannten Sensoren eignen sich für eine Vielzahl unterschiedlicher sensorbasierter Sortierverfahren und sind jeweils in unterschiedlichen Ausführungen mit jeweils unterschiedlichen Eigenschaften im Handel verfügbar. Somit ergibt sich durch die große Mehrzahl von Sensoren die Möglichkeit, mit dem erfindungsgemäßen Verfahren eine hervorragende Auswahl eines Sensors treffen zu können.

Gemäß einer bevorzugten weiteren Ausführungsform der vorliegenden Erfindung ist vorgesehen, dass die sensorbasierte Sortierung im Rahmen eines Verfahrens im Berg-und/oder Tagebau verwendet wird, und/oder wobei die sensorbasierte Sortierung im Rahmen eines Recyclingverfahrens verwendet wird und/oder wobei die sensorbasierte Sortierung im Rahmen eines Verfahrens zur Herstellung, Behandlung oder Verwertung von Lebensmitteln verwendet wird. Im Bergbau, in der Abfallwirtschaft und in der Lebensmittelindustrie werden sensorbasierte Sortierverfahren mit hohem Durchsatz eingesetzt. Die optimale Auswahl des Sensors ist hier von besonderer Bedeutung, da bereits kleine Ungenauigkeiten bei der Sortierung dazu führen können, dass das Sortierverfahren unwirtschaftlich wird.

Ein weiterer Gegenstand der vorliegenden Erfindung ist ein Verfahren zur sensorbasierten Sortierung eines Materialgemischs zur Konzentration eines Wertstoffes, wobei die Sortierung anhand einer Messung einer Materialeigenschaft mit einem Sensor durchgeführt wird, wobei der Sensor mit einem Verfahren nach einem der Ansprüche 1 bis 12 aus einer Mehrzahl von Sensoren ausgewählt wird.

Alle vorstehenden Ausführungen unter "Offenbarung der Erfindung" gelten gleichermaßen für das erfindungsgemäße Verfahren zur Auswahl eines Sensors und das erfindungsgemäße Verfahren zur sensorbasierten Sortierung.

Weitere Einzelheiten, Merkmale und Vorteile der Erfindung ergeben sich aus den Zeichnungen, sowie aus der nachfolgenden Beschreibung von bevorzugten Ausführungsformen anhand der Zeichnungen. Die Zeichnungen illustrieren dabei lediglich beispielhafte Ausführungsformen der Erfindung, welche den wesentlichen Erfindungsgedanken nicht einschränken.

### Kurze Beschreibung der Zeichnungen

- Figur 1: zeigt eine schematische Ansicht eines Verfahrens zur sensorbasierten Sortierung gemäß einer beispielhaften Ausführungsform der vorliegenden Erfindung.
- Figur 2: zeigt ein Ablaufschema eines Verfahrens zur Auswahl eines Sensors gemäß einer beispielhaften Ausführungsform der vorliegenden Erfindung.

### Ausführungsformen der Erfindung

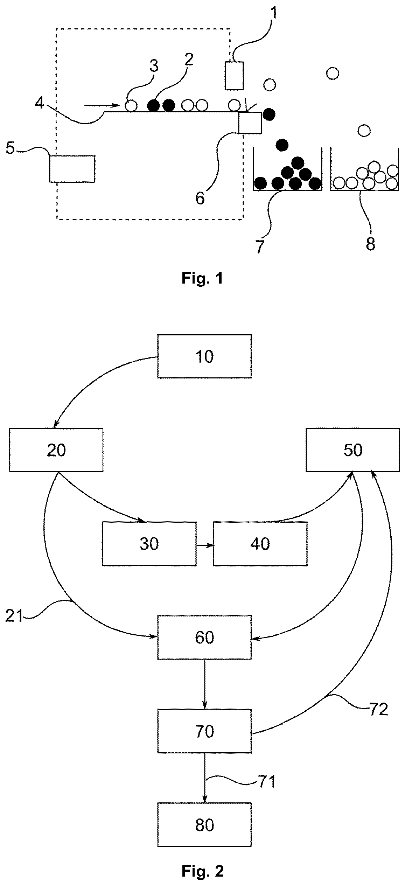

**Figur 1** zeigt eine schematische Ansicht eines Verfahrens zur sensorbasierten Sortierung gemäß einer beispielhaften Ausführungsform der vorliegenden Erfindung. Wertstoffhaltige Komponenten 2 und wertstofffreie Komponenten 3 eines Materialgemisches (nicht gezeigt) werden über einen Zulauf 4 der Sortierung zugeführt (angedeutet durch einen Pfeil). Der Sensor 1 detektiert kontaktlos eine Materialeigenschaft der zugeführten Komponenten 2, 3. Die vom Sensor 1 gewonnen Daten werden über eine Datenleitung (gestrichelte Linie) an einen Rechner 5 übermittelt, welcher entsprechend der vom Sensor 1 detektierten Materialeigenschaft entscheidet, ob eine Komponente eine wertstoffhaltige Komponente 2 oder eine wertstofffreie Komponente 3 ist. Auf Grundlage der gefällten Entscheidung steuert der Rechner einen Aktuator 6, welcher wertstoffhaltige Komponenten 2 dem Konzentrat 7 und wertstofffreie Komponenten 3 dem Haldenmaterial 8 zuführt. Dies wird beispielsweise durch einen gezielten Luftstoß vollzogen.

**Figur 2** zeigt ein Ablaufschema eines Verfahrens zur Auswahl eines Sensors gemäß einer beispielhaften Ausführungsform der vorliegenden Erfindung. Stellt sich die Aufgabe, ein Materialgemisch sensorbasiert zu sortieren, so ist es von äußerster Wichtigkeit, einen optimal an seine Aufgabe angepassten Sensor für das sensorbasierte Sortierverfahren zu finden. Aus dem, vorzugsweise bereits aufbereiteten, zu sortierenden Materialgemisch 10 werden repräsentative Einzelproben genommen 20. Das Materialgemisch kann beispielsweise aus Gesteinen aus dem Berg- oder Tagebau, aus zu sortierenden Materialien der Abfallwirtschaft oder aus Lebensmittelmaterial bestehen.

Die Einzelproben werden mechanisch behandelt und jeweils in einen Anschnitt und eine Restprobe zerteilt. Vorzugsweise werden dazu Einzelproben in Quader geformt, beispielsweise durch entsprechendes Sägen. Die Quader werden dann mit ihrer größten Fläche jeweils auf Glasplättchen geklebt und nah am Glasplättchen parallel zum Glasplättchen zerteilt. Der glasplättchenseitige Teil einer Einzelprobe ist der Anschnitt, der andere Teil ist die Restprobe. Die Anschnitte werden eingehend auf ihre jeweiligen Materialeigenschaften untersucht 30. Dies geschieht vorzugsweise mit elektronenmikroskopischen Untersuchungsmethoden, beispielsweise in einem Mineral Liberation Analyzer. Materialeigenschaften können beispielsweise der Mineralgehalt, Emissions- oder Absorptionsspektren im Optischen oder im Röntgenbereich, Dichte oder Magnetismus sein. Denkbar ist eine große weitere Vielzahl von physikalischen oder chemischen Materialeigenschaften.

Anschließend werden Sortierungen auf Grundlage jeweils unterschiedlicher Materialeigenschaften simuliert 40. Aus den simulierten Sortierungen wird die simulierte Sortierung herausgesucht, welche zu der höchsten Konzentration des Wertstoffes geführt hat. Anhand der Materialeigenschaft, welche dieser simulierten Sortierung zu Grunde lag, wird nun ein entsprechender Sensor ausgewählt 50.

Die Restproben der Einzelproben werden anschließend mit dem ausgewählten Sensor gemessen 21 und es erfolgt eine entsprechende Sortierung der Einzelproben 60 auf Grundlage der gemessenen Materialeigenschaft und eine Überprüfung der erfolgten Sortierung 70. Wenn die erfolgte Sortierung der der Einzelproben der simulierten Sortierung entspricht 71, so kann der ausgewählte Sensor in ein Verfahren zur sensorbasiertes Sortierung implementiert werden 80. Sollte die erfolgte Sortierung der der Einzelproben der simulierten Sortierung nicht entsprechen, so werden vorzugsweise Einstellungen des ausgewählten Sensors angepasst. Führt auch das nicht zu einer Übereinstimmung zwischen der erfolgten Sortierung und simulierten Sortierung 72, so wird eine andere Materialeigenschaft und/oder ein anderer Sensor gewählt 50 und die Restproben der Einzelproben werden anschließend mit dem anderen Sensor erneut vermessen und es erfolgt eine entsprechende Sortierung auf Grundlage der Messung mit dem anderen Sensor 60 beziehungsweise auf Grundlage der gemessenen anderen Materialeigenschaft. Stimmen die erfolgte Sortierung und die simulierte Sortierung wieder nicht überein, so kann der andere Sensor in seinen Einstellungen angepasst werden oder es werden ein weiterer anderer Sensor und/oder eine weitere andere Materialeigenschaft gewählt. Dies wird so oft durchgeführt, bis ein Sensor und eine zu detektierende Materialeigenschaft gewählt sind, welche optimale Ergebnisse bei der Sortierung ermöglichen.

### Bezugszeichenliste

- 1: Sensor
- 2: wertstoffhaltige Komponente
- 3: wertstofffreie Komponente
- 4: Zulauf
- 5: Rechner
- 6: Aktuator
- 7: Konzentrat
- 8: Haldenmaterial

- 10: zu sortierendes Materialgemisch
- 20: Auswahl repräsentativer Einzelproben
- 21: Messung der Restproben mit ausgewähltem Sensor
- 30: Messung der Materialeigenschaften der Einzelproben
- 40: Simulation einer Mehrzahl von Sortierungen
- 50: Auswahl eines Sensors
- 60: Sortierung der Einzelproben
- 70: Überprüfung der erfolgten Sortierung der Einzelproben
- 71: erfolgte Sortierung entspricht der simulierten Sortierung
- 72: erfolgte Sortierung entspricht nicht der simulierten Sortierung
- 80: Implementierung des Sensors

## Patentansprüche

1. Verfahren zur Auswahl eines Sensors (1) aus einer Mehrzahl von Sensoren zur sensorbasierten Sortierung eines Materialgemischs (10) anhand einer Materialeigenschaft zur Konzentration eines Wertstoffes, wobei in einem ersten Schritt aus dem Materialgemisch (10) repräsentative Einzelproben gewonnen werden (20),
in einem zweiten Schritt Materialeigenschaften der Einzelproben gemessen werden (30),
in einem dritten Schritt auf Grundlage der gemessenen Materialeigenschaften eine Mehrzahl von simulierten Sortierungen anhand jeweils unterschiedlicher Materialeigenschaften simuliert wird (40),
in einem vierten Schritt eine simulierte Sortierung mit einer hohen Wertstoffkonzentration aus der Mehrzahl von simulierten Sortierungen ausgewählt wird,
in einem fünften Schritt ein Sensor (1) zur Messung der Materialeigenschaft ausgewählt wird (50), welcher geeignet ist, mindestens eine der Materialeigenschaften, welche für die ausgewählte simulierte Sortierung genutzt wurde, zu messen.

2. Verfahren nach Anspruch 1, wobei die Einzelproben anhand von Messungen mit dem ausgewählten Sensor (1) sortiert werden und überprüft wird (60), ob die anhand der Messungen mit dem ausgewählten Sensor (1) erfolgte Sortierung der Einzelproben mit der ausgewählten simulierten Sortierung übereinstimmt (70).

3. Verfahren nach Anspruch 2, wobei, wenn die anhand der Messungen mit dem ausgewählten Sensor (1) erfolgte Sortierung der Einzelproben mit der simulierten Sortierung nicht übereinstimmt,
Einstellungen des ausgewählten Sensors (1) angepasst werden um die Sortierung der Einzelproben anhand von Messungen mit dem ausgewählten Sensor (1) mit der ausgewählten simulierten Sortierung zur Übereinstimmung zu bringen.

4. Verfahren nach einem der Ansprüche 2 oder 3, wobei, wenn es nicht möglich ist, die Sortierung der Einzelproben anhand von Messungen mit dem ausgewählten Sensor (1) mit der ausgewählte simulierten Sortierung in Übereinstimmung zu bringen (72), der vierte Schritt und der fünfte Schritt wiederholt werden und eine andere Materialeigenschaft und/oder ein anderer Sensor (1) gewählt wird.

5. Verfahren nach einem der vorhergehenden Ansprüche, wobei die Einzelproben in einem vorbereitenden Schritt zwischen dem ersten Schritt und dem zweiten Schritt mechanisch bearbeitet werden, wobei an den Einzelproben bevorzugt eine plane Seite hergestellt wird, wobei die Einzelproben besonders bevorzugt zu Quadern gesägt werden.

6. Verfahren nach Anspruch 5, wobei die Einzelproben jeweils mit der planen Seite auf ein Glasplättchen aufgeklebt werden, wobei die Einzelproben jeweils parallel zum Glasplättchen in einen glasplättchenseitigen Anschliff und eine Restprobe getrennt werden, wobei der Anschliff vorzugsweise als Gesteinsanschliff präpariert wird.

7. Verfahren nach Anspruch 6, wobei das Trennen in Anschliff und Restprobe in einem Abstand von 5 µm bis 1000 µm, bevorzugt von 50 µm bis 700 µm, besonders bevorzugt von 100 µm bis 400 µm und insbesondere von circa 200 µm von dem Glasplättchen durchgeführt wird.

8. Verfahren nach einem der Ansprüche 6 bis 7, wobei im zweiten Schritt zum Messen der Materialeigenschaften der Einzelproben (30) elektronenmikroskopische Untersuchungsmethoden verwendet werden, wobei vorzugsweise ein Rasterelektronenmikroskop mit einem energiedispersiven Spektrometer verwendet wird, wobei besonders bevorzugt als energiedispersives Spektrometer ein energiedispersives Röntgenspektrometer verwendet wird, wobei insbesondere zum Messen der Materialeigenschaften der Einzelproben (30) ein System zum automatisierten Analysieren von mineralogischen Proben, beispielsweise ein Mineral Liberation Analyzer, verwendet wird.

9. Verfahren nach einem der vorhergehenden Ansprüche, wobei im zweiten Schritt mit durch die Messungen der Materialeigenschaften der Einzelproben gewonnen Daten für die Einzelproben jeweils eine zweidimensionale Mineralverteilungskarte der Einzelprobe erstellt wird, wobei bevorzugt in den Mineralverteilungskarten jeweils Informationen zu Mineralgehalt der Einzelprobe und/oder zur Korngröße von Mineralen der Einzelprobe und/oder zu Verwachsungen der Minerale der Einzelprobe hinterlegt werden.

10. Verfahren nach einem der Ansprüche 6 bis 9, wobei die Messung der Einzelproben nach Anspruch 2 an den jeweiligen Restproben durchgeführt wird.

11. Verfahren nach einem der vorhergehenden Ansprüche, wobei als Sensor (1) der Mehrzahl von Sensoren zumindest ein Röntgensensor, vorzugsweise ein Dual-Energy X-Ray Transmission-Sensor und/oder ein Röntgenfloureszenz-Sensor, und/oder ein Farbsensor und/oder ein Infrarot-Sensor und/oder ein Magnet-Sensor und/oder ein Sensor zur Bestimmung elektrischer Felder verwendet wird.

12. Verfahren nach einem der vorhergehenden Ansprüche, wobei die sensorbasierte Sortierung im Rahmen eines Verfahrens im Berg- und/oder Tagebau verwendet wird, und/oder wobei die sensorbasierte Sortierung im Rahmen eines Recyclingverfahrens verwendet wird und/oder wobei die sensorbasierte Sortierung im Rahmen eines Verfahrens zur Herstellung, Behandlung oder Verwertung von Lebensmitteln verwendet wird.

13. Verfahren zur sensorbasierten Sortierung eines Materialgemischs (10) zur Konzentration eines Wertstoffes, wobei die Sortierung anhand einer Messung einer Materialeigenschaft mit einem Sensor (1) durchgeführt wird, wobei der Sensor (1) mit einem Verfahren nach einem der Ansprüche 1 bis 12 aus einer Mehrzahl von Sensoren ausgewählt wird.

## Claims

1. Method for selecting a sensor (1) from a plurality of sensors for sensor-based sorting of a material mixture (10) by way of a material property to concentrate a recyclable material, wherein
in a first step representative individual samples are obtained (20) from the material mixture (10),
in a second step material properties of the individual samples are measured (30),
in a third step, on the basis of the measured material properties, a plurality of simulated sortings is simulated (40) based on different material properties in each case, in a fourth step a simulated sorting having a high recyclable material concentration is selected from the plurality of simulated sortings,
in a fifth step a sensor (1), suitable for measuring at least one of the material properties used for the selected simulated sorting, is selected (50) for measuring the material property.

2. Method according to claim 1, wherein the individual samples are sorted by way of measurements with the selected sensor (1), and it is checked (60) whether the sorting of the individual samples which is performed using the measurements with the selected sensor (1) is in line (70) with the selected simulated sorting.

3. Method according to claim 2, wherein, if the sorting of the individual samples which is performed by way of the measurements with the selected sensor (1) is not in line with the simulated sorting,
settings of the selected sensor (1) are adjusted so as to bring the sorting of the individual samples by way of measurements with the selected sensor (1) in line with the selected simulated sorting.

4. Method according to either claim 2 or claim 3, wherein, if it is not possible to bring (72) the sorting of the individual samples by way of measurements with the selected sensor (1) in line with the selected simulated sorting, the fourth step and the fifth step are repeated and a different material property and/or a different sensor (1) are selected.

5. Method according to any of the preceding claims, wherein the individual samples are mechanically processed in a preparatory step between the first step and the second step, a planar face preferably being produced on the individual samples, the individual samples particularly preferably being sawn into cuboids.

6. Method according to claim 5, wherein the planar face of each of the individual samples is adhered to a glass platelet, the individual samples each being separated, parallel to the glass platelet, into a polished section on the glass platelet side and a sample remainder, the polished section preferably being prepared as a polished stone section.

7. Method according to claim 6, wherein the separation into a polished section and a sample remainder is performed at a distance of 5 µm to 1000 µm, preferably 50 µm to 700 µm, particularly preferably 100 µm to 400 µm and in particular approximately 200 µm from the glass platelet.

8. Method according to either claim 6 or claim 7, wherein in the second step electron-microscopy analysis methods are used for measuring the material properties of the individual samples (30), wherein preferably a scanning electron microscope having an energy-dispersive spectrometer is used, wherein particularly preferably an energy-dispersive X-ray spectrometer is used as an energy-dispersive spectrometer, wherein in particular a system for automated analysis of mineralogical samples, for example a mineral liberation analyser, is used for measuring the material properties of the individual samples (30).

9. Method according to any of the preceding claims, wherein in the second step, for each of the individual samples, a two-dimensional mineral distribution map of the individual sample is created using data obtained from the measurements of the material properties of the individual samples, the mineral distribution maps preferably each storing information on the mineral content of the individual sample and/or on the particle size of minerals in the individual sample and/or on intergrowths of the minerals in the individual sample.

10. Method according to any of claims 6 to 9, wherein the measurement of the individual samples according to claim 2 is carried out on the respective sample remainders.

11. Method according to any of the preceding claims, wherein at least one X-ray sensor, preferably a dual-energy X-ray transmission sensor and/or an X-ray fluorescence sensor, and/or a colour sensor and/or an infrared sensor and/or a magnet sensor and/or a sensor for determining electric fields, is used as the sensor (1) of the plurality of sensors.

12. Method according to any of the preceding claims, wherein the sensor-based sorting is used in the context of a mining or surface mining process, and/or wherein the sensor-based sorting is used in the context of a recycling process, and/or wherein the sensor-based sorting is used in the context of a process for producing, treating or exploiting foodstuffs.

13. Method for sensor-based sorting of a material mixture (10) to concentrate a recyclable material, wherein the sorting is carried out using a measurement of a material property with a sensor (1), the sensor (1) being selected from a plurality of sensors by a method according to any of claims 1 to 12.

## Revendications

1. Procédé pour sélectionner un capteur (1) parmi une pluralité de capteurs pour le tri par capteur d'un mélange de matériaux (10) sur la base d'une propriété de matériau pour concentrer un matériau valorisable, dans lequel dans une première étape, des échantillons individuels représentatifs sont obtenus (20) à partir du mélange de matériaux (10),
dans une deuxième étape, des propriétés de matériau des échantillons individuels sont mesurées (30),
dans une troisième étape, sur la base des propriétés de matériau mesurées, une pluralité de tris simulés est simulés en utilisant à chaque fois des propriétés de matériau différentes (40),
dans une quatrième étape, un tri simulé avec une concentration élevée de matériau valorisable est sélectionné parmi la pluralité de tris simulés,
dans une cinquième étape, un capteur (1) pour mesurer la propriété de matériau est sélectionné (50), lequel est approprié pour mesurer au moins une des propriétés de matériau qui a été utilisée pour le tri simulé sélectionné.

2. Procédé selon la revendication 1, dans lequel les échantillons individuels sont triés sur la base de mesures avec le capteur (1) sélectionné et il est vérifié (60) si le tri des échantillons individuels effectué sur la base des mesures avec le capteur (1) sélectionné correspond (70) au tri simulé sélectionné.

3. Procédé selon la revendication 2, dans lequel, lorsque le tri des échantillons individuels sur la base des mesures avec le capteur (1) sélectionné ne correspond pas au tri simulé,
des réglages du capteur (1) sélectionné sont adaptés pour faire correspondre le tri des échantillons individuels sur la base de mesures avec le capteur (1) sélectionné avec le tri simulé sélectionné.

4. Procédé selon l'une des revendications 2 ou 3, dans lequel, lorsqu'il n'est pas possible de faire correspondre (72) le tri des échantillons individuels sur la base de mesures avec le capteur (1) sélectionné avec le tri simulé sélectionné, la quatrième étape et la cinquième étape sont répétées et une autre propriété de matériau et/ou un autre capteur (1) sont sélectionnés.

5. Procédé selon l'une des revendications précédentes, dans lequel les échantillons individuels sont traités mécaniquement dans une étape préparatoire entre la première étape et la deuxième étape, dans lequel une face plane est de préférence produite sur les échantillons individuels, dans lequel les échantillons individuels sont, de manière particulièrement préférée, sciés en parallélépipèdes.

6. Procédé selon la revendication 5, dans lequel les échantillons individuels sont chacun collés par la face plane sur une plaquette de verre, dans lequel les échantillons individuels sont chaque fois séparés parallèlement à la plaquette de verre en une coupe polie du côté de la plaquette de verre et un échantillon résiduel, dans lequel la coupe polie est de préférence préparée sous la forme d'une coupe polie de roche.

7. Procédé selon la revendication 6, dans lequel la séparation en coupe polie et échantillon résiduel est effectuée à une distance de 5 µm à 1000 µm, de préférence de 50 µm à 700 µm, de manière particulièrement préférée de 100 µm à 400 µm et en particulier d'environ 200 µm de la plaquette de verre.

8. Procédé selon l'une des revendications 6 à 7, dans lequel, dans la deuxième étape, pour mesurer les propriétés de matériau des échantillons individuels (30), on utilise des méthodes d'examen par microscopie électronique, dans lequel on utilise de préférence un microscope électronique à balayage avec un spectromètre à dispersion d'énergie, de manière particulièrement préférée un spectromètre à rayons X à dispersion d'énergie, dans lequel, en particulier, on utilise pour mesurer les propriétés de matériau des échantillons individuels (30) un système d'analyse automatisée d'échantillons minéralogiques, par exemple un analyseur de libération minérale.

9. Procédé selon l'une des revendications précédentes, dans lequel, dans la deuxième étape, une carte de distribution des minéraux bidimensionnelle de l'échantillon individuel est créée pour chacun des échantillons individuels à partir des données obtenues par les mesures des propriétés de matériau des échantillons individuels, dans lequel, de préférence, des informations sur la teneur en minéraux de l'échantillon individuel et/ou sur la grosseur de grain des minéraux de l'échantillon individuel et/ou sur les imbrications des minéraux de l'échantillon individuel sont enregistrées dans chacune des cartes de distribution des minéraux.

10. Procédé selon l'une des revendications 6 à 9, dans lequel la mesure des échantillons individuels selon la revendication 2 est effectuée sur les échantillons résiduels respectifs.

11. Procédé selon l'une des revendications précédentes, dans lequel on utilise comme capteur (1) parmi la pluralité de capteurs au moins un capteur de rayons X, de préférence un capteur à transmission de rayons X à double énergie et/ou un capteur de fluorescence de rayons X, et/ou un capteur de couleurs et/ou un capteur infrarouge et/ou un capteur magnétique et/ou un capteur de détermination de champs électriques.

12. Procédé selon l'une des revendications précédentes, dans lequel le tri par capteur est utilisé dans le cadre d'un procédé d'exploitation minière et/ou d'exploitation à ciel ouvert, et/ou dans lequel le tri par capteur est utilisé dans le cadre d'un procédé de recyclage et/ou dans lequel le tri par capteur est utilisé dans le cadre d'un procédé de fabrication, de traitement ou de transformation de denrées alimentaires.

13. Procédé de tri par capteur d'un mélange de matériaux (10) pour concentrer un matériau valorisable, dans lequel le tri est effectué sur la base d'une mesure d'une propriété de matériau avec un capteur (1), dans lequel le capteur (1) est sélectionné parmi une pluralité de capteurs par un procédé selon l'une des revendications 1 à 12.
